# EUROPÄISCHE PATENTSCHRIFT

(11) **EP 0 427 161 B1**
(45) Veröffentlichungstag und Bekanntmachung des Hinweises auf die Patenterteilung: **20.04.1994**
(21) Anmeldenummer: 90121097.1
(22) Anmeldetag: 03.11.1990
(51) Int. Cl.: C07D 213/24, C07D 213/75, C07D 213/40, A61K 31/44

(54) **Picolylselenobenzamide von Aminopyridinen, Anilinen und Picolylaminen**
Picolylselenobenzamides of aminopyridines, anilines and picolylamines
Picolylsélénobenzamides d'aminopyridines, d'anilines et de picolylamines

(30) Priorität: 08.11.1989 DE 3937171
(43) Veröffentlichungstag der Anmeldung: 15.05.1991
(73) Patentinhaber: A. Nattermann & Cie. GmbH, D-50829 Köln (DE)
(72) Erfinder: Evers, Michel, Dr., B-4000 Liège (BE); Parnham, Michael, Dr., W-5024 Pulheim (DE); Römer, Axel, Dr., W-5030 Hürth-Gleuel (DE); Fischer, Hartmut, Dr., W-5000 Köln 41 (DE); Dereu, Norbert, Dr., W-5030 Hürth (DE)
(74) Vertreter: Döring, Wolfgang, Dr.-Ing. Patentanwälte Hauck, Graalfs, Wehnert, Döring, Siemons

(56) Entgegenhaltungen:
- DE-A- 3 443 467
- DE-A- 3 626 554

## Beschreibung

Die Erfindung betrifft neue Picolylselenobenzamide und ihre Salze, Verfahren und Zwischenprodukte zu ihrer Herstellung und diese Verbindungen enthaltende pharmazeutische Präparate.

DE-A-3443467 beschreibt S-(Carbamoylphenylselenyl)-Derivate. DE-A-3626554 beschreibt Diselenobis-benzoesäureamide.

Die erfindungsgemäßen Verbindungen entsprechen der allgemeinen Formel I 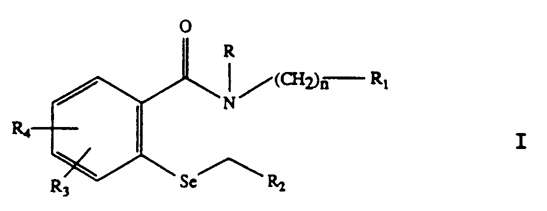
worin
- R: Wasserstoff, Ethyl oder Methyl bedeutet,
- R¹: für die Pyridylgruppe oder die substituierte Phenylgruppe steht, R² für die Pyridylgruppe steht und
- R³,R⁴: gleich oder verschieden sind und unabhängig voneinander für Wasserstoff, Fluor, Chlor, Brom, C₁₋₄-Alkyl, C₁₋₄-Alkoxy, Hydroxy, Cyano, Nitro oder zusammen für Methylendioxy stehen und
- n: Null oder 1 bedeutet.
Besonders bevorzugt sind dabei die Verbindungen, in denen
- n: gleich Null ist
- R: Wasserstoff bedeutet und
- R¹: für die Pyridylgruppe oder die substituierte Phenylgruppe steht, R² für die Pyridylgruppe steht und
- R³,R⁴: gleich oder verschieden sind und unabhängig voneinander für Wasserstoff, Fluor, Chlor, Brom, C₁₋₄-Alkyl, C₁₋₄-Alkoxy, Hydroxy, Cyano, Nitro oder zusammen für Methylendioxy stehen.

Die Verbindungen können auch in ihrer Salzform vorliegen.

Ebenfalls besonders bevorzugt sind die Verbindungen der allgemeinen Formel I, in der
- n: gleich 1 ist und
- R: für Wasserstoff steht, während
- R¹: für die Pyridylgruppe oder die substituierte Phenylgruppe steht, R² für die Pyridylgruppe steht und
- R³,R⁴: gleich oder verschieden sind und unabhängig voneinander Wasserstoff, Fluor, Chlor, Brom, C₁₋₄-Alkyl, C₁₋₄-Alkoxy, Hydroxy, Cyano oder Nitro bedeuten oder zusammen für Methylendioxy stehen.

Erfindungsgemäße Verbindungen sind beispielsweise:
2-(2-Picolylseleno)-N-(2-pyridyl)benzamid
2-(2-Picolylseleno)-N-(4-pyridyl)benzamid-dihydrochlorid
2-(2-Picolylseleno)-N-(4-pyridyl)benzamid
2-(3-Picolylseleno)-N-(2-pyridyl) benzamid
2-(4-Picolylseleno)-N-(2-pyridyl) benzamid
2-(4-Picolylseleno)-N-(3-pyridyl) benzamid
2-(4-Picolylseleno)-N-(4-pyridyl) benzamid
2-(2-Picolylseleno)-N-(3-pyridyl) benzamid-hydrochlorid
2-(2-Picolylseleno)-N-(3-pyridyl) benzamid-dihydrochlorid
2-(3-Picolylseleno)-N-(3-pyridyl) benzamid-dihydrochlorid
2-(3-Picolylseleno)-N-(4-pyridyl) benzamid-dihydrochlorid
2-(2-Picolylseleno)-N-(2-picolyl)benzamid
2-(2-Picolylseleno)-N-(3-picolyl) benzamid
2-(3-Picolylseleno)-N-(3-picolyl) benzamid
N-Phenyl-2-(2-picolylseleno) benzamid
N-Phenyl-2-(3-picolylseleno) benzamid
N-Phenyl-2-(4-picolylseleno) benzamid
N-(4-Chlorphenyl)-2-(4-picolylseleno)-benzamid
N-(4-Fluorphenyl)-2-(4-picolylselno)-benzamid

Die erfindungsgemäßen Verbindungen können über die isolierbaren 2-(Picolylseleno)-benzoesäuren hergestellt werden. Hierfür sind beispielsweise geeignet:
2-(2-Picolylseleno)-benzoesäure
2-(3-Picolylseleno)-benzoesäure
2-(4-Picolylseleno)-benzoesäure

Die 2-(Picolylseleno)-benzoesäuren können entweder durch Umsetzung mit Bis-[2-oxo-oxazolidinyl]phosphorylchorid und einem Anilin oder Pyridin-haltigen Amin in einem chlorierten Kohlenwasserstoff zu den erfindungsgemäßen Produkten führen (Reaktionsschema 1) oder die Umsetzung erfolgt mit 2-Chlor-1-methylpyridiniumjodid und einem Pyridin-haltigen Amin zum erfindungsgemäßen Produkt (Reaktionsschema 2).

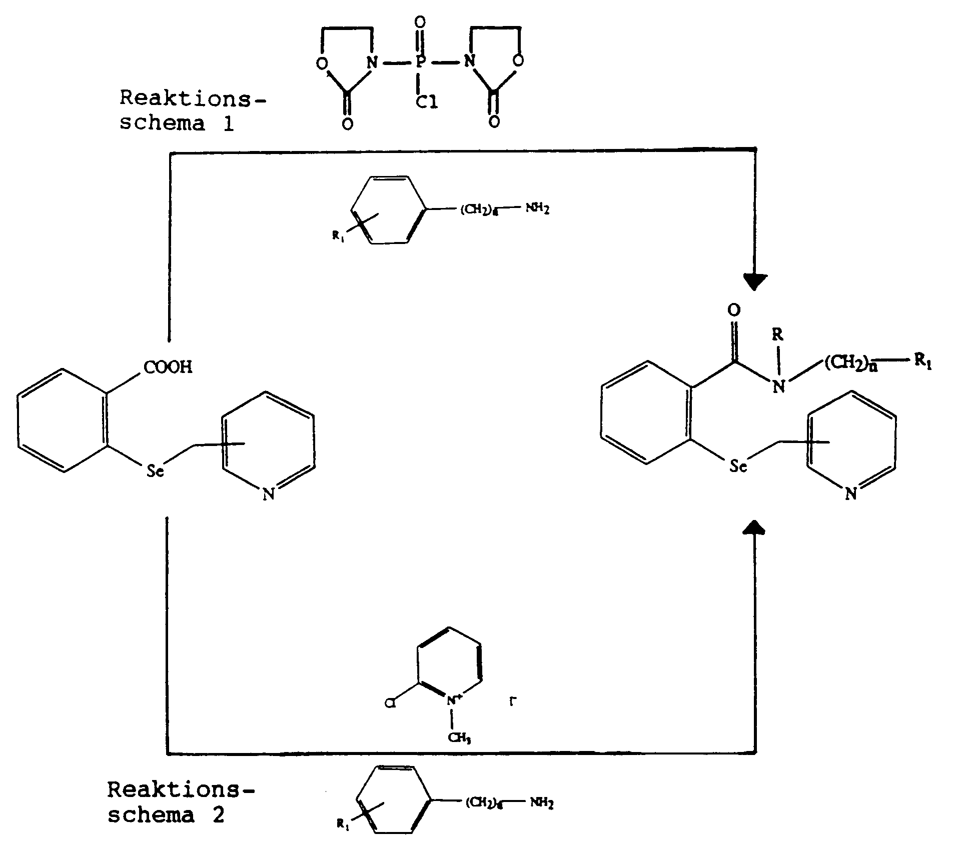

Die beanspruchten Verbindungen der Formel I können auch erhalten werden, in dem man die Picolylselenobenzoesäuren mit Chlormethylendimethyliminiumchlorid und einem Pyridin-haltigen Amin oder einen Anilin umsetzt und das Reaktionsprodukt aus dem Gemisch abtrennt:

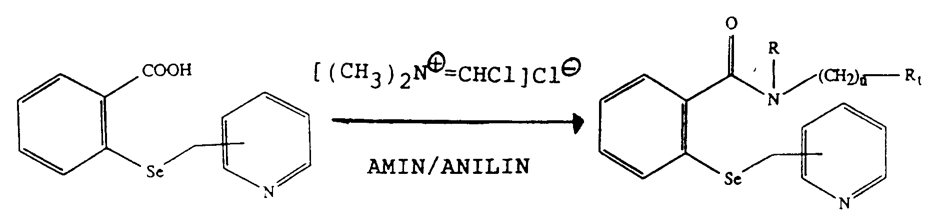

Die ebenfalls beanspruchten Picolylselenobenzoesäuren werden hergestellt, in dem man Diselenosalicylsäure mit Natriumdithionit in Natronlauge reduziert, dann mit Picolylchlorid-hydrochlorid in wäßriger Lösung umsetzt und das fertige Produkt aus dem Reaktionsgemisch abtrennt:

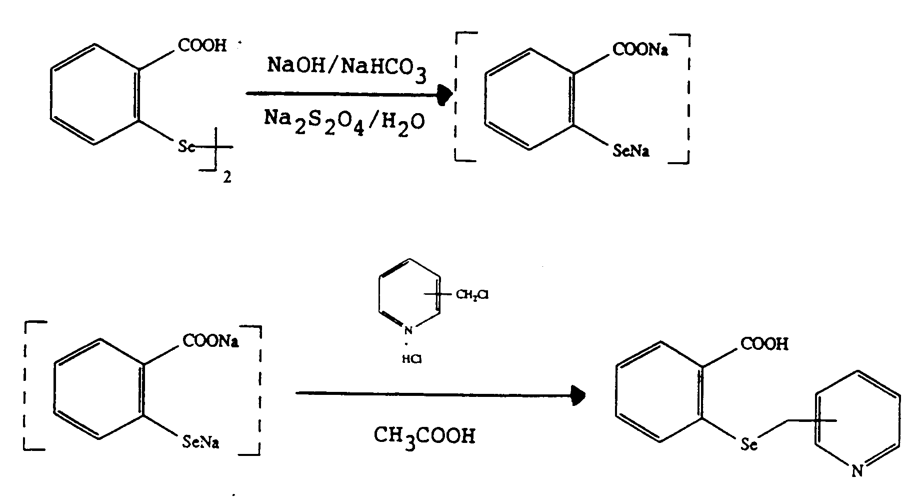

Die vorliegende Erfindung betrifft ebenfalls pharmazeutische Präparate, welche Verbindungen der Formel I als Wirkstoffe enthalten. Bei den erfindungsgemäßen pharmazeutischen Präparaten handelt es sich um solche zur enteralen wie oralen oder rektalen sowie parenteralen Verabreichung, welche die pharmazeutischen Wirkstoffe alleine oder zusammen mit einem üblichen, pharmazeutisch anwendbaren Trägermaterial enthalten. Vorteilhafterweise liegt die pharmazeutische Zubereitung des Wirkstoffes in Form von Einzeldosen vor, die auf die gewünschte Verabreichung abgestimmt sind, wie z.B. Tabletten, Dragées, Kapselen, Suppositorien, Granulate, Lösungen, Emulsionen oder Suspensionen. Die Dosierung der Substanz liegt üblicherweise zwischen 10 und 1000 mg pro Tag, vorzugsweise zwischen 30 und 300 mg pro Tag, und kann in einer Dosis oder mehreren Teildosen, vorzugsweise in zwei bis drei Teildosen pro Tag, verabreicht werden.

Es konnte festgestellt werden, daß ein derartiges pharmazeutisches Präparat hervorragende entzündungshemmende Eigenschaften besitzt.

Die Herstellung der erfindungsgemäßen Verbindungen wird durch die folgenden Beispiele näher erläutert. Die angegebenen Schmelzpunkte wurden mit einem Büchi-510-Schmelzpunktbestimmungsapparat gemessen und sind mit °C angegeben und nicht korrigiert.

### Beispiel 1

### 2-(2-Picolylseleno)-benzoesäure

Zu einer Suspension von 10 g (0,025 Mol) Diselenosalicylsäure in 75 ml Wasser wird unter Rühren 7,6 g (0,19 Mol) Natriumhydroxid zugegeben, wobei die Innentemperatur leicht ansteigt und die Säure in Lösung geht. Anschließend werden 20 g (0,189 Mol) Natriumcarbonat und 11,6 g (0,067 Mol) Natriumdithionit unter leichter Erwärmung zugegeben, dann 2 h am Rückflußkühler erhitzt. Nach Abkühlen auf Raumtemperatur werden innerhalb 10 Minuten 9,8 g (0,06 Mol) 2-Picolylchlorid-hydrochlorid, gelöst in 20 ml Wasser, zugetropft und 14 h bei Raumtemperatur weitergerührt. Anschließend wird mit 25%iger Salzsäure auf pH 8 bis 9 und dann mit Essigsäure (98%ig) auf pH 5-6 eingestellt. Das ausgefallene weiße Rohprodukt wird abgesaugt, mit Wasser neutral gewaschen und aus 2-Propanol (900 ml) umkristallisiert.
Ausbeute: 10,5 g (71,9% d.Th.)
Fp.: 220-221°C

### Beispiel 2

### 2-(3-Picolylseleno)-benzoesäure

Analog Beispiel 1 erhält man unter Verwendung von 9,8 g (0,06 Mol) 3-Picolylchlorid-hydrochlorid als Produkt 2-(3-Picolylseleno) benzoesäure, die nach der Umsetzung aus 660 ml Methanol umkristallisiert wird.
Ausbeute: 9,5g (65 % d.Th.)
Fp.: 237°C

### Beispiel 3

### 2-(4-Picolylseleno)-benzoesäure

Analog Beispiel 1 erhält man unter Verwendung von 9,8g (0,06 Mol) 4-Picolylchlorid-hydrochlorid als Produkt 2-(4-Picolylseleno)-benzoesäure. Die Umkristallisation erfolgt aus 250 ml Methanol.
Ausbeute: 10,8g (74 % d.Th.)
Fp.: 243-245 °C

### Beispiel 4

### 2-(2-Picolylseleno)-N-(2-pyridyl)benzamid

Zu einer auf 0°C abgekühlten Lösung von 5,84 g (0,02 Mol) 2-(2-Picolylseleno)-benzoesäure (aus Beispiel 1) in 40 ml Dichlormethan werden auf einmal 5 g (0,02 Mol) Bis-[2-oxo-3-oxazolidinyl]phosphorylchlorid gegeben. Nach 2 h Rühren bei Raumtemperatur werden bei 10°C nacheinander
1,88 g (0,02 Mol) 2-Aminopyridin,
50 ml Dichlormethan und
6,4 ml Triethylamin zugetropft und 20 h bei Raumtemperatur gerührt. Es wird 2 mal mit je 100 ml H₂O ausgeschüttelt, über Natriumsulfat getrocknet und eingeengt. Das braune Öl (7 g) wird in 100 ml 1 N HCl aufgenommen, filtriert und das Filtrat mit 13 ml 10 N NaOH alkalisch gestellt und mit 100 ml Essigester extrahiert. Die organische Phase wird getrocknet, eingeengt und der so erhaltene feste Rückstand 48 h mit 100 ml Diethylether ausgerührt. Das abfiltrierte Rohprodukt wird durch Säulenchromatographie (Kieselgel / Dichlormethan) gereinigt und aus 20 ml 2-Propanol umkristallisiert.
Ausbeute: 1,0 g (13,6% d.Th.)
Fp.: 139-140°C

### Beispiel 5

### 2-(2-Picolylseleno)-H-(4-pyridyl)benzamid-dihydrochlorid

Eine Lösung von 4 g (0,0137 Mol) 2-(2-Picolylseleno)benzoesäure aus Beispiel 1 und 2 ml Triethylamin in 40 ml Dichlormethan wird mit 3,5 g (0,0137 Mol) 2- Chlor-1-methylpyridiniumjodid versetzt und 1 Stunde bei Raumtemperatur gerührt. Dann wird innerhalb 15 Minuten eine Lösung von 1,29 g (0,0137 Mol) 4-Aminopyridin und 2 ml Triethylamin in 30 ml Dichlormethan zugetropft und nach 2 Stunden Rühren der Kolbeninhalt mit 100 ml 1 N NaOH ausgeschüttelt, dann 2 mal mit je 100 ml Wasser. Die organische Phase wird getrocknet und eingeengt und der Rückstand in 2-Propanol (80 ml)/15%ige HCl (10 ml) aufgenommen. Das abfiltrierte feste Rohprodukt wird in 100 ml Methanol gelöst und solange Diethylether zugetropft, bis die Lösung trüb bleibt. Nach 2 Stunden werden die gebildeten Kristalle abfiltriert und getrocknet.
Ausbeute: 3,9 g (70,9% d.Th.)
Fp.: 228-230° C

### Beispiel 6

### 2-(2-Picolylseleno)-N-(4-pyridyl)benzamid

Eine Lösung von 0,5 g (0,00113 Mol) 2-(2-Picolylseleno)-N-(4-pyridyl)benzamid-dihydrochlorid aus Beispiel 5 in 2 ml H₂O wird mit 10 M NaOH alkalisch gestellt und 2 mal mit je 5 ml Dichlormethan extrahiert. Die organische Phase wird über Magnesiumsulfat getrocknet und eingeengt und das Rohprodukt aus 15 ml 2-Propanol umkristallisiert.
Ausbeute: 0,40 g (96% d.Th.)
Fp.: 136 - 137°C

### Beispiel 7

### 2-(2-Picolylseleno)-N-(3-pyridyl)-benzamid-hydrochlorid

In eine auf -10°C abgekühlte Lösung von 3 g (0,0103 Mol) 2-(2-Picolylseleno)-benzoesäure (aus Beispiel 1) in 20 ml Dichlormethan werden unter Rühren 1,32 g (0,0103 Mol) Chlormethylen-dimethyliminium-chlorid eingetragen, 2 Stunden bei Raumtemperatur gerührt und dann innerhalb 10 Minuten eine Lösung von 0,97 g (0,0103 Mol) 3-Aminopyridin und 2,32 g (0,023 Mol) Triethylamin in 20 ml Dichlormethan zugetropft und 14h bei Raumtemperatur gerührt, mit 50 ml Wasser ausgeschüttelt und die abgetrennte Dichlormethanphase getrocknet und eingeengt. Der ölige Rückstand wird mit 50 ml 1 N HCl versetzt und man filtriert, danach wird mit NaOH (5%ig) alkalisch gestellt und 2 mal mit je 50 ml Dichlormethan ausgeschüttelt. Die über Natriumsulfat getrocknete organische Phase wird eingeengt, der Rückstand in 40 ml 2-Propanol gelöst und unter Rühren langsam mit 15 ml 2-Propanol/10%ige HCl versetzt; dabei fällt das Hydrochlorid kristallin aus. Es wird mit 50 ml Diethylether nachgewaschen.
Ausbeute: 1,85 g (44,4 d.Th.)
Fp.: 221 - 222°C

### Beispiel 8

### 2-(2-Picolylseleno)-N-(3-picolyl)-benzamid-dihydrochlorid

Analog Beispiel 7 erhält man aus 3,0 g (0,0103 Mol) 2-(2-(3-Picolylseleno)-benzoesäure (Beispiel 1) und 1,11 g (0,0103 Mol) 3-Picolylamin als Produkt 2-(2-Picolylseleno)-N-(3-picolyl)-benzamid-dihydrochlorid
Ausbeute: 3,1 g (66,3 % d.Th.)
Fp.: 193-195° C

### Beispiel 9

### 2-(3-Picolylseleno)-benzoesäure-N-(4-pyridyl) benzamid-dihydrochlorid

Zu einer auf 0°C abgekühlten Lösung von 4 g (0,0132 Mol) 2-(3-Picolylseleno)benzoesäure (Beispiel 2) und 4,4 ml Triethylamin in 40 ml Dichlormethan werden 3,5 g (0,0132 Mol) Bis-(2-oxo-3-oxazolidinyl)phosphorylchlorid auf einmal gegeben. Nach 15 Minuten werden noch 1,3 g (0,0138 Mol) 4-Aminopyridin zugesetzt und das Gemisch 3,5 h bei 0 °C gerührt. Dann wird 2 mal mit je 100 ml Wasser ausgeschüttelt, die organische Phase über Natriumsulfat getrocknet und eingeengt. Der Rückstand wird in 60 ml 2-Propanol gelöst und mit 10 ml 15%iger HCl-Lösung (in 2-Propanol) versetzt. Das so entstandene feste Produkt wird in 50 ml H₂O gelöst und mit 1 g Aktivkohle versetzt, 15 Minuten bei Raumtemperatur gerührt und dann filtriert. Zum Filtrat wird Aceton zugetropft, bis die Lösung trüb bleibt. Nach 2 h werden die gebildeten Kristalle abfiltriert und getrocknet.
Ausbeute: 1,3 g (21,51 d.Th.)
Fp.: 249-254° C

### Beispiel 10

### 2-(3-Picolylseleno)-N-(3-pyridyl)benzamid-dihydrochlorid

Eine Suspension von 4 g (0,0137 Mol) 2-(3-Picolylseleno)benzoesäure aus Beispiel 2 in 40 ml Dichlormethan wird bei + 5°C auf einmal unter Rühren mit 1,76 g (0,0137 Mol) Chlormethylendimethyliminiumchlorid (Vilsmeier Reagenz) versetzt und 2 Stunden bei Raumtemperatur gerührt. Dann wird eine Lösung von 1,29 g (0,0137 Mol) 3-Aminopyridin und 3,2 ml Triethylamin in 30 ml Dichlormethan zugetropft und 2 h gerührt. Es wird mit 100 ml Dichlormethan verdünnt und mit 2 mal je 100 ml Wasser ausgeschüttelt, die organische Phase über Natriumsulfat getrocknet, eingeengt und der ölige Rückstand in 140 ml 2-Propanol gelöst. Die Lösung wird mit 10 ml 15%iger HCl (in 2-Propanol) aufgenommen, das gewonnene Rohprodukt 30 Minuten mit 20 ml 1 N NaOH gerührt und 2 mal mit je 100 ml Essigester ausgeschüttelt. Die organische Phase wird mit 200 ml Wasser gewaschen, getrocknet und eingeengt. Der viskose Rückstand wird in 100 ml 2-Propanol aufgenommen und mit 10 ml 15%iger HCl (in 2-Propanol) versetzt. Das feste Rohprodukt wird in 100 ml Methanol gelöst und solange Diethylether zugetropft, bis die Lösung trüb bleibt. Nach 2 h werden die ausgefallenen Kristalle abgesaugt und getrocknet.
Ausbeute: 2,3 g (41% d.Th.)
Fp.: 261 - 263°C

### Beispiel 11:

### N-Phenyl-2-(2-picolylseleno)benzamid

Zu einer auf 0-5° C abgekühlten Lösung von 7 g (0,024 Mol) 2-(2-Picolylseleno)-benzoesäure (aus Beispiel 1) und 2,4,g (0,024 Mol) Triethylamin in 40 ml Dichlormethan gibt man in fester Form und auf einmal 6g(0,024 Mol) Bis(2-oxo-3-oxazolidinyl)phosphorylchlorid.

Nach 20 Minuten Rühren wird innerhalb von 5 Minuten eine Lösung von 2,2 g (0,024 Mol) Anilin in 5 ml Dichlormethan und eine Lösung von 2,4 g (0,024 Mol) Triethylamin in 5 ml Dichlormethan zugetropft und nach 16 h bei Raumtemperatur das Reaktionsgemisch mit 100 ml Wasser gewaschen. Die organische Phase wird nacheinander mit 100 ml 10 % iger NaHCO₃ - Lösung, 100 ml 10 % iger Zitronensäure und 100ml Wasser ausgeschüttelt. Die organische Phase wird über Natriumsulfat getrocknet und im Wasserstrahlvakuum aufkonzentriert. Das feste Rohprodukt (6,3g) wird aus 35 ml 2-Propanol umkristalisiert.
Ausbeute: 4,8 g (54,4 % d. Th.)
Fp.: 135,5-136° C

### Beispiel 12:

### N-Phenyl-2-(3-picolylseleno)benzamid

Analog Beispiel 11 erhält man unter Verwendung von 7g (0,024 Mol) 3-Picolylseleno-benzoesäure (aus Beispiel 2) ein Rohprodukt, das durch Säulenchromatographie gereinigt wird (Kieselgel; Dichlormethan:Methanol 99 : 1; Fraktionen von je 50 ml).

Die Fraktionen 48 bis 128 werden im Wasserstrahlvakuum aufkonzentriert und das feste Rohprodukt aus 50 ml 2-Propanol umkristallisiert.
Ausbeute: 4,5g (51% d.Th.
Fp.: 136 - 137°C

### Beispiel 13:

### N-Phenyl-2-(4-picolylseleno)benzamid

Analog Beispiel 11 erhält man unter Verwendung von 7 g (0,024 Mol) 4-Picolylselenobenzoesäure (Beispiel 3) ein Rohprodukt, das durch Säulenchromatographie gereinigt wird (Kieselgel; Dichlormethan: Methanol 99:1; Fraktionen von je 50 ml).

Die Fraktionen 74 bis 104 werden in Wasserstrahlvakuum aufkonzentriert und das feste Rohprodukt aus 50 ml 2-Propanol umkristallisiert.
Ausbeute: 3,6 g (41 % d. Th.)
Fp.: 166 - 167°C

### Beispiel 14

### N-(4-Chlorphenyl)-2-(4-picolylseleno)benzamid

Analog Beispiel 11 erhält man unter Verwendung von 3,1 g (0,024 Mol) 4-Chloranilin ein Rohprodukt, das mit einer Mischung aus 10 ml 2-Propanol und 5 ml n-Hexan ausgerührt wird und 2 mal aus je 30 ml 2-Propanol umkristallisiert wird.
Ausbeute: 1,7g (18% d. Th.)
Fp.: 157-158° C

### Beispiel 15:

### N-(4-Fluorphenyl)-2-(4-picolylseleno)benzamid

Analog Beispiel 11 erhält man unter Verwendung von 2,7 g (0,024 Mol) 4-Fluoranilin ein festes Rohprodukt, das 2 mal aus je 30 ml 2-Propanol umkristalisiert wird.
Ausbeute: 2,6 g (28 % d. T.h.)
Fp.: 152,5-153,5° C

## Patentansprüche

1. Picolylselenobenzamide und ihre Salze der allgemeinen Formel I 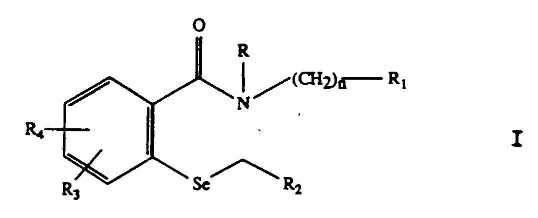 worin
R Wasserstoff, Methyl oder Ethyl bedeutet
R¹ für die Pyridylgruppe oder die substituierte Phenylgruppe steht, R² für die Pyridylgruppe steht und
R³,R⁴ gleich oder verschieden sind und unabhängig voneinander für Wasserstoff, Fluor, Chlor, Brom, C₁₋₄-Alkyl, C₁₋₄-Alkoxy, Hydroxy, Cyano, Nitro oder zusammen für Methylendioxy stehen und
n Null oder eins bedeutet.

2. Picolylselenobenzamide und ihre Salze der allgemeinen Formel I, Anspruch 1, worin
n gleich Null ist
R Wasserstoff bedeutet und
R¹ für die Pyridylgruppe oder substituierte Phenylgruppe steht, R² für die Pyridylgruppe steht und
R³,R⁴ gleich oder verschieden sind und unabhängig voneinander für Wasserstoff, Fluor, Chlor, Brom, C₁₋₄-Alkyl, C₁₋₄-Alkoxy, Hydroxy, Cyano, Nitro oder zusammen für Methylendioxy bedeuten.

3. Picolylselenobenzamide und ihre Salze der allgemeinen Formel I, Anspruch 1, worin
n gleich 1 ist
R Wasserstoff bedeutet und
R¹ für die Pyridylgruppe oder substituierte Phenylgruppe steht, R² für die Pyridylgruppe steht und
R³,R⁴ gleich oder verschieden sind und unabhängig voneinander für Wasserstoff, Fluor, Chlor, Brom, C₁₋₄Alkyl, C₁₋₄-Alkoxy, Hydroxy, Cyano, Nitro oder zusammen für Methylendioxy bedeuten.

4. 2-(2-Picolylseleno)benzoesäure

5. 2-(3-Picolylseleno)-benzoesäure

6. 2-(4-Picolylseleno)-benzoesäure

7. Verfahren zur Herstellung von Verbindungen nach den Ansprüchen 1 bis 3, dadurch gekennzeichnet, daß man in einem chlorierten Kohlenwasserstoff eine nach Anspruch 4 bis 6 beanspruchte Verbindung mit Bis[2-oxo-3-oxazolidinyl]phosphorylchlorid und einem Anilin oder Pyridin-haltigen Amin umsetzt und das Produkt aus dem Reaktionsgemisch abtrennt.

8. Verfahren zur Herstellung von Verbindungen nach den Ansprüchen 1 bis 3, dadurch gekennzeichnet, daß man in einem chlorierten Kohlenwasserstoff eine der in Anspruch 4 bis 6 genannten Verbindungen mit 2-Chlor-1-methylpyridiniumjodid und einem Anilin oder Pyridin-haltigen Amin umsetzt und das Produkt aus dem Reaktionsgemisch abtrennt.

9. Verfahren zur Herstellung von Verbindungen nach den Ansprüchen 1 bis 3, dadurch gekennzeichnet, daß man in einem chlorierten Kohlenwasserstoff eine der in Anspruch 4 bis 6 genannten Verbindungen mit Chlormethylen-dimethyliminiumchlorid und einem Anilin oder Pyridin-haltigen Amin umsetzt und das Produkt aus dem Reaktionsgemisch abtrennt.

10. Verfahren zur Herstellung der Verbindung nach Anspruch 4, dadurch gekennzeichnet, daß man Diselenosalicylsäure mit Natriumdithionit in Natronlauge reduziert, dann mit Picolylchloridhydrochlorid in wäßriger Lösung umsetzt und das fertige Produkt nach Aufarbeitung aus dem Reaktionsgemisch abtrennt.

11. Pharmazeutische Präparate, dadurch gekennzeichnet, daß sie entzündungshemmende Eigenschaften besitzen und als Wirkstoff eine Verbindung der Formel I gemäß den Ansprüchen 1 bis 3 im Gemisch mit üblichen pharmazeutischen Hilfs- und Trägerstoffen enthalten.

## Claims

1. Picolylselenobenzamides and their salts with the general formula I 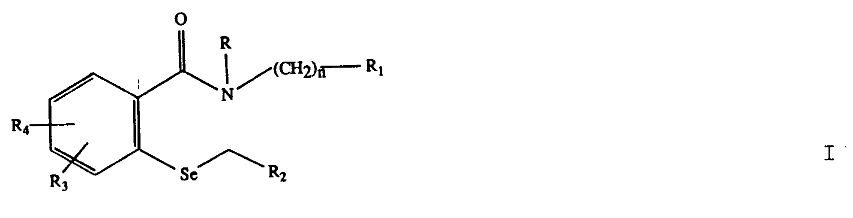 where
R is hydrogen, methyl or ethyl
R¹ represents the pyridyl group or substituted phenyl group, R² the pyridyl group and
R³, R⁴ are identical or different and, taken separately, represent hydrogen, fluorine, chlorine, bromine, C₁₋₄-alkyl, C₁₋₄-alkoxy, hydroxy, cyano or nitro or, taken together, methylenedioxy and
n is zero or one.

2. Picolylselenobenzamides and their salts according to general formula I, claim 1, where
n equals zero
R is hydrogen and
R¹ represents the pyridyl group or substituted phenyl group, R² the pyridyl group and
R³, R⁴ are identical or different and, taken separately, represent hydrogen, fluorine, chlorine, bromine, C₁₋₄-alkyl, C₁₋₄-alkoxy, hydroxy, cyano or nitro, or taken together, methylenedioxy.

3. Picolylselenobenzamides and their salts according to general formula I, claim 1, where
n equals 1
R is hydrogen and
R¹ represents the pyridyl group or substituted phenyl group, R² the pyridyl group and
R³, R⁴ are identical or different and, taken separately, represent hydrogen, fluorine, chlorine, bromine, C₁₋₄-alkyl, C₁₋₄-alkoxy, hydroxy, cyano or nitro or, taken together, methylenedioxy.

4. 2-(2-Picolylseleno)benzoic acid

5. 2-(3-Picolylseleno)benzoic acid

6. 2-(4-Picolylseleno)benzoic acid

7. Method of preparation of compounds according to claims 1 to 3 wherein one of the compounds named in claims 4 to 6 is reacted in a chlorinated hydrocarbon with bis [2-oxo-3-oxazolidinyl]phosphoryl chloride and an aniline or pyridine-containing amine and the product is isolated from the reaction mixture.

8. Method of preparation of compounds according to claims 1 to 3 wherein one of the compounds named in claims 4 to 6 is reacted in a chlorinated hydrocarbon with 2-chloro-1-methylpyridinium iodide and an aniline or pyridine-containing amine and the product is isolated from the reaction mixture.

9. Method of preparation of compounds according to claims 1 to 3 wherein one of the compounds named in claims 4 to 6 is reacted in a chlorinated hydrocarbon with chloromethylenedimethyliminium chloride and an aniline or pyridine-containing amine and the product is isolated from the reaction mixture.

10. Method of preparation of the compound according to claim 4, wherein diselenosalicylic acid is reduced with sodium dithionite in caustic soda solution, then reacted with picolyl chloride hydrochloride in aqueous solution and the finished product isolated from the reaction mixture after work-up.

11. Pharmaceutical products with antiinflammatory properties, which contain as an active ingredient a compound of formula I according to claims 1 to 3 in admixture with usual pharmaceutical additives and excipients.

## Revendications

1. Picolylsélénobenzamides et leurs sels, de la formule générale I 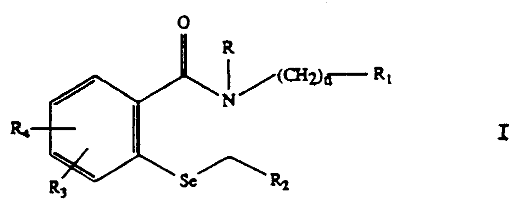 dans laquelle
R représente un atome d'hydrogène, le radical méthyle ou éthyle,
R¹ représente le radical pyridyle ou un radical phényle substitué, R² représente le radical pyridyle et
R³, R⁴, qui peuvent avoir des significations identiques ou différentes et qui représentent, chacun indépendamment l'un de l'autre, un atome d'hydrogène, de fluor, de chlore, de brome, un radical alkyle en C₁-C₄, alcoxy en C₁-C₄, hydroxyle, cyano, nitro, ou forment ensemble un radical méthylènedioxy et
n est égal à zéro ou à un.

2. Picolylsélénobenzamides et leurs sels, de la formule générale I, suivant la revendication 1, caractérisés en ce que
n est égal à zéro,
R représente un atome d'hydrogène et
R¹ représente le radical pyridyle ou un radical phényle substitué, R² représente le radical pyridyle et
R³, R⁴, qui peuvent avoir des significations identiques ou différentes et représentent, chacun indépendamment l'un de l'autre, un atome d'hydrogène, de fluor, de chlore, de brome, un radical alkyle en C₁-C₄, alcoxy en C₁-C₄, hydroxyle, cyano, nitro, ou forment ensemble un radical méthylènedioxy.

3. Picolylsélénobenzamides et leurs sels, de la formule générale I suivant la revendication 1, caractérisés en ce que
n est égal à 1,
R représente un atome d'hydrogène et
R¹ représente le radical pyridyle ou un radical phényle substitué, R² représente le radical pyridyle et
R³, R⁴, qui peuvent avoir des significations identiques ou différentes et qui représentent, chacun indépendamment l'un de l'autre, un atome d'hydrogène, de fluor, de chlore, de brome, un radical alkyle en C₁-C₄, alcoxy en C₁-C₄, hydroxyle, cyano, nitro, ou forment ensemble un radical méthylènedioxy.

4. Acide 2-(2-picolylséléno)-benzoïque.

5. Acide 2-(3-picolylséléno)-benzoïque.

6. Acide 2-(4-picolylséléno)-benzoïque.

7. Procédé de préparation de composés suivant l'une quelconque des revendications 1 à 3, caractérisé en ce que l'on fait réagir un composé revendiqué dans l'une quelconque des revendications 4 à 6 avec le chlorure de bis[2-oxo-3-oxazolidinyl]phosphoryle et une aniline ou une amine contenant de la pyridine, dans un hydrocarbure chloré et on sépare le produit du mélange réactionnel.

8. Procédé de préparation de composés suivant l'une quelconque des revendications 1 à 3, caractérisé en ce que l'on fait réagir l'un des composés cités dans les revendications 4 à 6 avec l'iodure de 2-chloro-1-méthylpyridinium et une aniline ou une amine contenant de la pyridine, dans un hydrocarbure chloré et on sépare le produit du mélange réactionnel.

9. Procédé de préparation de composés suivant l'une quelconque des revendications 1 à 3, caractérisé en ce que l'on fait réagir l'un des composés cités dans les revendications 4 à 6 avec le chlorure de chlorométhylène-diméthyliminium et une aniline ou une amine contenant de la pyridine, dans un hydrocarbure chloré et on sépare le produit du mélange réactionnel.

10. Procédé de préparation du composé suivant la revendication 4, caractérisé en ce que l'on fait réagir l'acide disélénosalicylique avec le dithionite de sodium dans une solution aqueuse d'hydroxyde de sodium, puis avec le chlorhydrate du chlorure de picolyle en solution aqueuse et on sépare le produit prêt du mélange réactionnel après traitement.

11. Préparations pharmaceutiques, caractérisées en ce qu'elles possèdent des propriétés anti-inflammatoires ou antiphlogistiques et contiennent, à titre de principe actif, un composé de la formule I suivant l'une quelconque des revendications 1 à 3, en mélange à des excipients, véhicules et adjuvants pharmaceutiques usuels.
